# EUROPEAN PATENT APPLICATION

(11) **EP 1 520 580 A1**
(43) Date of publication of application: **06.04.2005**
(21) Application number: 04256094.6
(22) Date of filing: 01.10.2004
(51) Int. Cl.: A61K 31/198, A61P 3/00, A61P 43/00

(54) **Creatine composition and use**

(30) Priority: 03.10.2003 GB 0323240
(71) Applicant: Golini, Jeffrey M., Billings, Montana (US)
(72) Inventor: Golini, Jeffrey M., Billings, Montana (US)
(74) Representative: Brown, David Leslie

(57) **Abstract**

The invention relates to the preparation and use of compositions for increasing endurance in a mammal and the treatment prevention and delaying of endurance fatigue. The compositions contain creatine and an alkaline substance. The compositions also have use in the reduction of the perception of fatigue during or after endurance exercise.

## Description

### FIELD OF THE INVENTION

The present invention relates to an oral creatine supplement, and the use of this for increasing endurance and delaying or decreasing endurance fatigue.

### BACKGROUND OF THE INVENTION

Taking creatine orally has been used to increase creatine and creatine phosphate stores in the human body. This is important for athletes requiring explosive energy because creatine aids in the process of creating energy usable by muscles of the athlete. Sports in which creatine is used include weightlifting and sprinting.

When an athlete exercises or tenses a muscle, energy is required for the muscle to function properly. The energy it uses comes from several different sources, but primarily from nutrients obtained from food. These nutrients are broken down by natural processes occurring within the human body, and new compounds formed which are used to develop energy used by muscles. One of these compounds is adenosine triphosphate (ATP). When muscle energy is needed this ATP is broken down one step further into a chemical called adenosine diphosphate (ADP). This process releases energy which is then used by the contracting muscles. Without sufficient ATP, muscles do not perform properly.

Known energy increasers and stimulants have only superficially energized the body, and do not increase the body's ability to produce its own ATP stores.

Muscle can store only limited amounts of ATP. As a result, it has been found that with about 5-10 seconds of muscle exertion, the amount of stored ATP is depleted. This results in muscle failure and fatigue. When this happens, the body tries to restore its immediate source of ATP by borrowing a high energy phosphate from a chemical called creatine phosphate (CP). Muscle cells store the chemical, CP, in the same way it stores ATP. If high intensity exercise goes beyond 10 seconds, the body will continue to try and restore its ATP levels by a process called glycolysis. This process is complicated and is a slow method of restoring ATP levels. This is a special problem for anaerobic athletes who require instant energy to maintain and sustain high powered muscle contractions.
By orally supplementing with creatine, an athlete can enhance his body's storage levels of CP. As the muscle runs out of ATP, it can recharge itself by borrowing this CP molecule. Research has shown that by supplementing with 5 grams of creatine, 4-6 times a day, for two or more days, the human body showed a significant increase in total creatine concentration.

ATP or CP cannot be ingested directly by athletes because these chemicals are destroyed by the digestive system of the athlete. However, it has been found that creatine can be ingested and converted by the body to CP. The resulting cellular concentrations of creatine after administration, is stable and is not prone to dissipation.

The most commonly used oral creatine supplement is creatine monohydrate. The most commonly used amounts have varied from 20 to 30 grams daily. It has been taken in powder, capsule, tablet and liquid form. The creatine is mixed with or taken with water, fruit juice, acidic effervescent drink or acidic fruit flavored drinks.

As well as creatine monohydrate, other forms of creatine have also been used, such as creatine citrate and also creatine pyruvate. These other forms of creatine are administered similar to the method of administrating creatine monohydrate.

The main problem with conventional creatine supplementation is the ability to deliver creatine in a usable form by the human body. Research has shown that known creatine delivery systems actually have the human body ingesting creatinine, a poison and toxic byproduct. It is believed that the main reason for complaints resulting from creatine consumption, namely, stomach cramps, edema, bloodedness and dehydration, is caused by the body's defence to this toxic compound.

Oral creatine supplements are usually dissolved in acidic solutions having a pH range of from 3-6. Research has shown that at these pH levels, the rate of conversion of creatine to creatinine is almost instantaneous.

US Patent 6,399,661 discloses an oral creatine supplement and the method of making this supplement which includes mixing an alkaline powder with a powdered creatine until the pH of the mixture is in the range between 7-14. A powdered additive is added to the mixture for improving sweetness and taste. Finally, a further alkaline powder is added to the mixture to adjust the pH of the mixture to a range between 7-14. This mixture is then mixed with water prior to ingestion. This supplement is advantageous because of substantially decreased creatinine formation relative to that in more acidic products.

Alkaline powders that may be used include a hydroxide, carbonate, bicarbonate, chloride, tree latex or a phosphate. Magnesium glyceryl phosphate and soda ash are preferred, especially soda ash.

The creatine powder may be comprised of creatine monohydrate, creatine phosphate, creatine pyruvate, or creatine citrate.

The mixed powder may be ingested in a number of forms. It may be ingested following mixing with water.

Alternatively it may be ingested as a capsule. A flow agent (eg magnesium stearate) may be added to the mixed powder and the resulting mixture may be encapsulated.

In a further alternative, the ingested material may be in the form of a tablet containing a hardener material (eg sorbitol), a binder material (eg microcrystalline cellulose), and a flow agent (eg magnesium stearate), may be combined with the mixture and compressed into tablets.

In a further alternative prepared by adding water to the mixture together with a creatine solution the base material (eg glycerine) and a stabilizer material (eg potassium sorbate).

In a further alternative the process ingested material is a soft gel. A gel base material (eg soy bean oil) to the mixture for forming the softgel.

Creatine is known for increasing power or explosive muscle activity and for increasing muscle bulk. Creatine has not previously been found to be beneficial for endurance activity and athletes using vigorous muscle activity for more than 90 seconds, often more than 4 minutes and often of several hours duration.

### DISCLOSURE OF THE INVENTION

In one aspect the invention provides a method of increasing endurance in a human or non-human mammal, which comprises administering to the mammal a composition or compositions comprising creatine and an alkaline substance wherein the pH of the composition is in the range 7-14.

The term "increased (or "increasing") endurance" refers to enhanced performance or decreased fatigue during vigorous muscle activity sustained for at least 90 seconds, often at least 4 minutes and sometimes for hours. Cycling and running are examples of activities using vigorous muscle activity. The term "vigorous muscle activity" not only relates to cycling and running but also other types of activity characterized by frequent onset of fatigue over the period 90 seconds to 3 hours and continuous exercise. Race walking and swimming are further examples.

The alkali may be a hydroxide, carbonate, bicarbonate, chloride, tree latex or a phosphate.

Preferred akalis are magnesium glyceryl phosphate and sodium bicarbonate (soda ash), particularly soda ash.

The creatine is preferably in a form suitable for ingestion such as creatine monohydrate, creatine phosphate, creatine pyruvate and creatine citrate. The creatine and alkali may be in an aqueous solution, a capsule, a tablet or a soft gel.

Preferably the creatine and alkali are prepared by mixing an alkaline powder with a powdered creatine to form a mixture with a pH in the range 7-14, adding a powder that is additive to the mixture for improving sweetness and taste and if necessary further alkaline powder to adjust the pH of the mixtures to the range 7-14.

In another aspect the invention provides use of creatine and an alkaline powder in the preparation of compositions for treating, preventing or delaying endurance fatigue. The compositions may take any of the forms described above including the forms disclosed in US patent 6,399,661.

The term "endurance fatigue" refers to fatigue resulting from muscle activity sustained for more than 90 seconds. Fatigue induced by running, cycling and other endurance sports is intended to be included by the meaning of the term "endurance fatigue". The term "fatigue" is defined as that the state of increased discomfort and decreased efficiency resulting from prolonged or excessive muscle activity; loss of power or capacity to respond to stimulation. Endurance fatigue is distinguishable from fatigue from explosive exercise in that the exercise causing endurance fatigue takes place for longer than 90 seconds. Commonly the exercise causes exhaustion following vigorous exercise taking more than 4 minutes and often for times greater than 10 minutes.

The preferred method of the invention is a non-therapeutic method, by which is intended to mean - in the case of a human subject - typically self-administration of the composition(s) without medical supervision or administration of the composition(s) by a person other than a qualified medical practitioner without medical supervision, the human subject being physically well and not suffering from a disease or other adverse medical condition. In the case of a non-human mammalian subject such as a companion-, guide-, working- or performing animal (e.g. a horse, pony, donkey, mule, ass, cow or dog), the non-therapeutic administration is intended to mean typically administration of the composition(s) by a controller or keeper of the animal, being a person other than a qualified medical practitioner and without medical supervision, the animal subject being physically well and not suffering from a disease or other adverse medical condition.

While creatine is known for increasing power or explosive muscle activity and for increasing muscle bulk, it has not proven beneficial for endurance athletes. Surprisingly in combination with alkali, endurance is dramatically improved. The effect is not simply due to the alkali as alkalis such as sodium bicarbonate, sodium phosphate and potassium phosphate are relatively ineffective for increasing endurance by themselves and are unpalatable. While the applicant does not wish to be bound by theory, his current thinking is that creatine acts as a delivery mechanism to get alkali into the muscle where it neutralizes lactic acid generated in muscles. The presence of creatine also appears to make increase the acceptability of the alkali by making it more palatable.

Five preferred separate systems have been developed for delivery of creatine in an oral supplement.

The first is a powder mix having the formulation in Table 1:

**TABLE 1**

| Powder | |
|---|---|
| Creatine Monohydrate Sugar (from dextrose, fructose, | 5000 mg |
| sucrose, or a type of sugar) | 10-15 g |
| Maltodextrin (from corn and/or rice) | 5-10 g |
| Soda Ash | 50 mg-1.5 g |
| Natural and/or Artificial Flavors | 2 g |
| Magnesium Glycerol Phosphate | 500 mg |
| Aspartame, Acesulfame K, Sucralose | |
| and/or Stevia Extract | 200 mg |
| (Powder mix can be with/without | |
| flavors, sugars or sweeteners) | |

The method for preparing the powder mix includes placing the 5000 mg of creatine monohydrate in a mixing vessel. The soda ash is then added to adjust the pH of the mixture from 7-14. After the pH has been adjusted, the sugar, natural and/or artificial flavors, and the aspartame, acesulfame K, sucralose and/or stevia extract are added to adjust the mixture for desired taste and desired sweetness. The pH of the mixture is again checked and magnesium glycerol phosphate is added to adjust the pH to be between 7 and 14. The powder is then bottled for distribution.
The second is the capsule having the formulation of Table 2:

**TABLE 2**

| Capsule | |
|---|---|
| Creatine Monohydrate | 1000 mg |
| Maltodextrin | 200 mg |
| Magnesium Stearate | 5 mg |
| Magnesium Glycerol Phosphate | 25 mg |
| Soda Ash | 5-1000 mg |
| Natural and/or Artificial Flavors | 20 mg |

The method for making capsules is to place 1000 mg of creatine monohydrate in a mixing vessel. The pH is adjusted to be between 7 and 14 by adding soda ash. The maltodextrin, magnesium stearate (a flow agent) and natural and/or artificial flavors are added to desired taste and sweetness. The pH is again checked, and magnesium glycerol phosphate is added to adjust the pH to be between 7 and 14. The mixed powder is then processed by a conventional encapsulation machine which prepares capsules of the powder. A capsule with this formulation is swallowed by a user and dissolves in the solution present in the stomach.

The third is the tablet formulation of Table 3:

**TABLE 3**

| Tablet | |
|---|---|
| Creatine Monohydrate | 250 mg |
| Sorbitol | 400 mg |
| Microcrystalline Cellulose | 50 mg |
| Magnesium Stearate | 5 mg |
| Magnesium Glycerol Phosphate | 25 mg |
| Soda Ash | 5-500 mg |

In preparing the tablets, creatine monohydrate is placed in a mixing vessel and soda ash is added to adjust the pH from between 7 and 14. Sorbitol, which is a hardener, and microcrystalline cellulose, which is a binder, is added to the mixture, as well as magnesium stearate, which is a stabilizer, in preparation for forming tablets. The pH of the mixture is then checked and magnesium glycerol phosphate is then added to adjust the pH to be between 7 and 14. The powder is then processed by a conventional machine which compresses the powder into tablets.

A tablet with this formulation is swallowed by a user and dissolves in the solution present in the stomach.

The fourth is the liquid formulation of Table 4:

**TABLE 4**

| Liquid Formulation | |
|---|---|
| Creatine Monohydrate | 3 grams |
| Water | 1-30 ml |
| Glycerine | 1-30 ml |
| Magnesium Glycerol Phosphate | 25 mg |
| Natural and/or Artificial Flavors | 5 ml |
| Soda Ash | 50-1000 mg |
| Potassium Sorbate | 200 mcg |

In preparing the liquid form, water and creatine monohydrate are mixed together in a mixing vessel. Soda ash is added to adjust the pH to be between 7 and 14. The glycerine, which acts as a base and preservative, together with potassium sorbate, which acts as a stabilizer and preservative, are added to the mixture. Further, natural and/or artificial flavors are added to adjust the mixture for desired taste and sweetness. The pH is again checked, and magnesium glycerol phosphate is then added to adjust the pH to be between 7 and 14. The resulting liquid is then bottled for distribution.

With this liquid form, the product is ingested directly. The fifth is the softgel form shown in Table 5:

**TABLE 5**

| Softgel Form | |
|---|---|
| Creatine Monohydrate | 100 mg |
| Sugar | 300 mg |
| Chocolate | 50 mg |
| Soy Bean Oil | 500 mg |
| Magnesium Glycerol Phosphate | 25 mg |
| Soda Ash | 5-1000 mg |

The method for making the softgel includes placing the creatine monohydrate in a mixing vessel. Soda ash is then added to adjust the pH to be between 7 and 14. Next, the sugar, chocolate and soy bean oil, which acts as a base for the gel, is added to the mixture. Again, the pH is checked, and magnesium glycerol phosphate is added to adjust the pH to be between 7 and 14. The resulting gel is then placed in bottles for distribution.

In these formulations, the pH of the solution is above 7, and the creatine formation is low relative to that at lower pH value.

It should be understood that these five formulations are among many that can be used. For example, organic or inorganic substances could be used with equally beneficial results to raise the pH of the solution. Hydroxides, carbonates, bicarbonates, chlorides, tree latex or phosphates could be used.

Further, the creatine used could be creatine monohydrate as described in the above formulations, or could be creatine phosphate, creatine pyruvate or creatine citrate.

The types, combination and amounts of buffers can vary with various delivery forms, flavors, and combination type products.

The method for enhancing a stable concentration of creatine in a human includes dissolving the creatine powder into water or any other type of fluid. Once the mixture has been mixed, the solution is ingested immediately and an effective amount of creatine is absorbed. The capsule, tablet and liquid form can be ingested as is.

This buffered delivery system enhances the delivery of usable creatine to the person taking the supplement, and overcomes the problems caused when creatine is converted to creatinine. The higher the pH, the more creatine a human will ingest.

In a further aspect the invention provides a method for reducing the perception of fatigue in a subject during or after endurance exercise comprising administering to the subject a composition or compositions comprising creatine and an alkaline substance wherein the pH of the composition is in the range 7-14.

In yet a further aspect the invention provides the use of creatine and an alkaline substance in the preparation of compositions for reducing the perception of fatigue in a subject during or after endurance exercise.

All the specific details, examples and preferences expressed herein for the composition(s) and administration in relation to the method of increasing endurance are equally applicable to the method for reducing the perception of fatigue after endurance exercise, and vice versa.

The compositions may be administered before, during or after exercise. It is currently preferred to administer the composition or compositions before exercise to provide decreased perception of fatigue and greater endurance during exercise.

### EXAMPLES

### EXAMPLE 1 - Benefit to long distance cyclists.

A cyclist ingested 5 grams of the powder of Table 1 mixed with water 15 minutes before a four hour cycle ride. The cyclist reported a boost in the endurance capacity for the first three hours of the ride. Subsequently the same dose of powder was administered to other cyclists who reported a similar boost of endurance capacity in three hour cycling events.

### EXAMPLE 2 - Five Minute Time Trials

Buffered creatine monohydrate was administered to an elite cyclist prior to time trials. A trial was conducted once a week. The trial was a 5 minute standing start full all out ride. The first week he used nothing so we had a benchmark to get a figure to establish if any improvement was being observed.

The distance covered in the 5 minutes was measured so that improvement could be measured. The dose was taken just one hour prior to the test with water. The material administered was that of Table 1. The results are shown in Table 6.

**TABLE 6**

| | Buffered creatine | Distance Covered | % improvement | Heart Rate |
|---|---|---|---|---|
| Week One | 0g | 6.14km | - | 178 |
| Week Two | 5g | 6.68 | 8.8% | 174 |
| Week Three | 7.5g | 6.82 | 11% | 176 |

### EXAMPLE 3 - Effects observed in Football Players

Buffered creatine was administered to groups of football players to ascertain its effect on endurance without any changes to diet, workout, or activity levels.

### Test Group 1

Group 1 was composed of professional male football players who are currently involved in a heavy workout schedule (pre-season), with all being seasoned veterans to this beginning part of the season. Their positions were wide receivers and defensive backs. The body weight for this group at the beginning of camp was as follows:

| | |
|---|---|
| Subject A | 185 lbs |
| Subject B | 187 lbs |
| Subject C | 190 lbs |
| Subject D | 195 lbs |

Group 1 started and finished with two 750mg capsules of Kre-Alkalyn^{R} (creatine 750 mg, soda ash 25 mg) which were administered in the morning. This went on 7 days a week for 6 weeks. Body fat, body weight, strength, endurance and stamina were measured before starting Kre-Alkalyn^{R}. Any aches and pains were also noted.

### Test Group 2

Group 2 was composed of professional male football players who are currently involved in a heavy workout schedule (pre-season), with all being seasoned veterans to this beginning part of the season. Their positions were running back and linebacker. The body weight for this group at the beginning of camp was as follows:

| | |
|---|---|
| Subject E | 225 lbs |
| Subject F | 227 lbs |
| Subject G | 235 lbs |
| Subject H | 237 lbs |

Group 2 started and finished with two 750mg capsules of Kre-Alkalyn^{R}, which were administered in the morning. This went on 7 days a week for 6 weeks. Body fat, body weight, strength, endurance and stamina were measured before starting Kre-Alkalyn^{R}. Any aches and pains were also noted.

### Test Group 3

Group 3 was composed of professional male football players who are currently involved in a heavy workout schedule (pre-season), with all being seasoned veterans to this beginning part of the season. Their positions were offensive lineman and defensive lineman. The body weight for this group at the beginning of camp was as follows:

| | |
|---|---|
| Subject E | 315 lbs |
| Subject F | 330 lbs |
| Subject G | 340 lbs |
| Subject H | 380 lbs |

Group 3 started with two 750 mg capsules of Kre-Alkalyn^{R}, for the first week, increasing to four 750 mg capsules at week 2 and remaining on that amount. Capsules were administered in the morning. This went on 7 days a week for 6 weeks. Body fat, body weight, strength, endurance and stamina were measured before starting Kre-Alkalyn^{R}. Any aches and pains were also noted.

### The Results

After a full 6 weeks of testing, the following were the results reported.

### Test Group 1

Overall energy levels seemed to have increased, with a total body fat % being lowered by 1%. All reported increased endurance levels through out the two a day (times 3 hour) practices. Recuperation between practices increased along with strength levels. Group 1 outperformed, out endured and outplayed other athletes of the same position throughout the 6 week period.

### Test Group 2

Overall energy levels seemed to have increased, with a total body fat % being lowered by 2%. All reported increased endurance levels through out the two a day (times 3 hour) practices. Recuperation between practices increased along with strength levels. Group 2 outperformed, out endured and outplayed other athletes of the same position throughout the 6 week period.

### Test Group 3

Overall energy levels seemed to have increased, with a total body fat % being lowered by 3.5%. All reported increased endurance levels through out the two a day (times 3 hour) practices. Recuperation between practises increased along with strength levels. Group 3 outperformed, out endured and outplayed other athletes of the same position throughout the 6 week period.

All three groups reported less aches and pains then they other athletes on the team. Each subject reported feeling better, had more energy, more endurance, more stamina than ever in the past.

From the testing and research done with the product Kre-Alkalyn, so we conclude that this product is very effective for increasing endurance and stamina, while eliminating fatigue due to lactic acid build up.

These 12 subjects are professional athletes who were into pre-season training and are very in tuned with their bodies.

### EXAMPLE 4 - Effects observed in Rats

White male albino rats where used for this study. A control group was used along with a test group. Upon arrival to the lab both groups entered Phase I, stabilization. Stabilization started with changing their diet to a more conventional food source. Good protein and carbohydrate levels, low fat. Each rat was weighed at the start and each Monday for six weeks. Rats were randomly selected for test group and control group.

Rats were observed for energy levels daily. Energy levels were measured by amount of chewing, maze work & cage activity that occurred.

Rats were placed in a large 10 gallon glass cages that allowed movement and building of nests. Chew items were also put in the cages to help measure night activity.

Kre-Alkalyn Administration: Based on human studies, 1.5 grams per 100 kg of body weight was used. This dose was given daily in oral form. This worked out to 15 mcg/gram of rat starting weight.

### The Start:

Both rats weighed in at 250 grams. Activity and Energy levels were extremely low. Neither rats would work a maze. During stabilization, both control rat and test rat sleep during the day, with very low night activity.

The results are shown in Table 7

**TABLE 7**

| **Animal** | **Weight in grams** | **Week Gain** | **Total Gain** | %Gained |
|---|---|---|---|---|
| Control | 250.00 | Starting Weight | | |
| Week 1 | 259.20 | 9.20 | 9.20 | 3.68 |
| Week 2 | 285.80 | 26.60 | 35.80 | 14.32 |
| Week 3 | 317.80 | 32.00 | 67.80 | 27.12 |
| Week 4 | 344.80 | 27.80 | 95.60 | 37.92 |
| Week 5 | 359.80 | 15.00 | 110.60 | 43.92 |
| Week 6 | 382.80 | 23.00 | **133.60** Total | **53.12** Total |

| Test | 250.00 | Starting Weight | | |
|---|---|---|---|---|
| Week 1 | 262.80 | 12.80 | 12.80 | 5.12 |
| Week 2 | 280.80 | 18.00 | 30.80 | 12.24 |
| Week 3 | 309.80 | 29.60 | 60.40 | 23.92 |
| Week 4 | 358.56 | 48.76 | 109.16 | 43.42 |
| Week 5 | 381.00 | 22.44 | 131.60 | 52.40 |
| Week 6 | 407.80 | 26.80 | **158.40** Total | **63.12** Total |

<
- Control Rat Weight Gain:: Although the control rat gain 133.6 grams, he was very round and soft.
- Test Rat Weight Gain:: Kre-Alkalyn Rat gained 158.4 grams or 63.12 % increase in lean muscle in 6 weeks. He gained 10% more than control rat. Test rat was solid with no roundness or flabbiness like the control rat.
- Control Rat Energy:: Control rat was not active at all during the day. He slept in his nest. The nest was a mess and un-organized. Chew toys were not touched. Rat did not even come out for feeding. The only evidence of night activity was food eaten. Control rat had no energy or motivation to work a maze.
- Test Rat Energy: Test rat was out of his nest each morning and most of the time during the day. His next was clean and very organized. Control rat went through 3 chew toys during the 6 week testing, along with chewing at the top of the cage which was only accessible when water bottle was climbed, which he did often during the day. Test rat was extremely motivated and energized to work a maze. His energy levels were extremely high for a rat.
- Conclusion:: Not only did the Test rat gain 63.12% lean muscle, but his energy levels was increased 100% over the control rat and 100% from the stabilization week.
Kre-Alkalyn (Buffered Creatine) increased lean muscle mass by 63.12% after 6 weeks, and energy levels were increases 100%..

### EXAMPLE 5 - Effects on Healthy Adults

Two subjects were administered 750 mg daily and two subjects were administered 1500 mg daily for 30 days. Kre-Alkalyn administration was taken first thing in the morning upon awaking. pH, Energy, Endurance & Physical levels were measured 3 times daily. The first in the A.M. before Kre-Alkalyn administration, the second mid day, and the 3^{rd} in the evening. The chart shown in Table 8 was used for measurements.

**TABLE 8**

| **Energy** | **Endurance** | **Physical** |
|---|---|---|
| 1-Very energetic | 1-Very | 1- Feel Great |
| 2- Fairly | 2-Good | 2-Good |
| 3-So So | 3-So So | 3-So So |
| 4-A bit sluggish | 4-Not so good | 4-Not so good |
| 5-No energy | 5-Bad | 5-Bad |

pH was measured by uranalyses. The subjects were asked to not change their diet or workout schedules. All 4 subjects were male from ages 25-44.

The following definitions were used for Energy, Endurance and Physical.

### ENERGY

1). A: dynamic quality (narrative energy) B: the capacity of acting or being active (intellectual energy) C: a usually positive spiritual force (the energy flowing through all people)
2). Vigorous exertion of power: EFFORT (investing time and energy)
3). A fundamental entity of nature that is transferred between parts of a system in the production of physical change within the system and usually regarded as the capacity for doing work 4), usable power (as heat or electricity); also : the resources for producing such power

### ENDURANCE

1). Permanence, duration
2). The ability to withstand hardship or adversity; especially :the ability to sustain a prolonged stressful effort or activity (a marathon runner's endurance)
3). The act or an instance of enduring or suffering
4). Capacity to endure pain or hardship, fortitude, stamina

### PHYSICAL

1). A emotional state or action
2). The overall quality of ones awareness and well being
3). Strength and power

### Results:

| Subject 1 | **Energy** | | |
|---|---|---|---|
| | Before study | 3.00 (So-So) | |
| | During study | 1.17 (Average) | Very Energetic |

| | **Endurance** | | |
|---|---|---|---|
| | Before study | 3.00 (So-So) | |
| | During study | 1.56 (Average) | Very |

| | **Physical** | | |
|---|---|---|---|
| | Before study | 2.00 (Good) | |
| | During study | 1.33 (Average) | Feel Great |
| | | | |

| Subject 2 | **Energy** | | |
|---|---|---|---|
| | Before study | 3.00 (So-So) | |
| | During study | 1.26 (Average) | Very Energetic |

| | **Endurance** | | |
|---|---|---|---|
| | Before study | 3.00 (So-So) | |
| | During study | 1.30 (Average) | Very |

| | **Physical** | | |
|---|---|---|---|
| | Before study | 3.00 (Good) | |
| | During study | 1.33 (Average) | Feel Great |
| | | | |

| Subject 3 | **Energy** | | |
|---|---|---|---|
| | Before study | 4.00 (A bit sluggish) | |
| | During study | 1.64 (Average) | Very Energetic |

| | **Endurance** | | |
|---|---|---|---|
| | Before study | 4.00 (Not so good) | |
| | During study | 1.57 (Average) | Very |

| | **Physical** | | |
|---|---|---|---|
| | Before study | 4.00 (Not so good) | |
| | During study | 1.76 (Average) | Feel Great |
| | | | |

| Subject 4 | **Energy** | | |
|---|---|---|---|
| | Before study | 5.00 (No energy) | |
| | During study | 3.76 (Average) So-So | |

| | **Endurance** | | |
|---|---|---|---|
| | Before study | 5.00 (Bad) | |
| | During study | 3.03 (Average) | So-So |

| | **Physical** | | |
|---|---|---|---|
| | Before study | 4.00 (Not so good) | |
| | During study | 3.06 (Average) | So-So |

### % Increases:

| | | | |
|---|---|---|---|
| Subject 1 | Energy | 156.4% | |
| | Endurance | 92.3% | |
| | Physical | 50.3% | |
| | | | |
| Subject 2 | Energy | 138.9% | |
| | Endurance | 130.7% | |
| | Physical | 125.5% | |
| | | | |
| Subject 3 | Energy | 143.9% | |
| | Endurance | 154.7% | |
| | Physical | 127.2% | |
| | | | |
| Subject 4 | Energy | 32.9% | |
| | Endurance | 65.1% | |
| | Physical | 30.7% | |

### Average for study % Increases:

| | |
|---|---|
| Energy | 118.0% |
| Endurance | 110.7% |
| Physical | 83.4% |

### Conclusion:

Kre-Alkalyn increased Energy levels by 118%, Endurance & Stamina by 110.7% and physical well being 83.4%.

### EXAMPLE 6 - Computerized bicycle testing

6 subjects were administered 1.5 grams of Kre-Akalyn in the morning. and 6 subjects were administered 1.5 grams of a placebo in the morning for 8 weeks. No changes were made to diet or off season training.

Endurance & Stamina levels were tested at the beginning of the study and every 2 weeks for 8 weeks.

The testing equipment used were Life Fitness computerized bicycles. These systems are able to monitor heat beat and revolutions per minute.

The pre test results showed that both the placebo group and the Kre-Alkalyn group were all at about the same Endurance & Stamina levels. They were barely able to work up and maintain a level 5, at 100 rpm's for 20 minutes.
- Placebo Group:: Not much progress was made from Week 1 to Week 8. At the end of week 8, placebo group could work up and maintain a level 6, at 100 rpm's for 20 minutes.
Stamina was still at 20 minutes while endurance was increased slightly to level 6.
- Kre-Alkalyn Group:: The results showed a very large effect. At the end of Week 8, Kre-Alkalyn group was able to work up to and maintain a level 15 at 100 rpm's for 40 minutes.
Stamina increases from 20 minutes to 40 minutes. A 100% increase. Endurance increased from level 5 to level 15 a 200% increase.
In conclusion, Kre-Alkalyn dramatically increases endurance and stamina in endurance athletes. Stamina increases of 100% and Endurance increases of 200%.

While the fundamental novel features of the invention have been illustrated in the above examples, it should be understood that various substitutions, modifications and variations may be made by those skilled in the art without departing from the spirit or scope of the invention. For example, different doses and alkaline compounds may be used. Accordingly, all such modifications or variations are included in the scope of the invention.

## Claims

1. A method of increasing endurance in a human or non-human mammal, which comprises administering to the mammal a composition or compositions comprising creatine and an alkaline substance wherein the pH of the composition is in the range 7-14.

2. A method as claimed in claim 1 wherein the alkaline substance is selected from a hydroxide, carbonate, bicarbonate, chloride, tree latex or a phosphate.

3. A method as claimed in claim 3, wherein the alkaline substance is selected from magnesium glyceryl phosphate and soda ash.

4. A method as claimed in claim 3 wherein the alkaline substance is soda ash.

5. A method as claimed in any one of claims 1-3 wherein the creatine is administered is creatine monohydrate, creatine phosphate, creatine pyruvate or creatine citrate.

6. A method as claimed in any one of claims 1-5 wherein the creatine and alkaline substance are administered as an aqueous solution, a capsule, a tablet or a soft gel.

7. A method as claimed in any one of claims 1-6 wherein a composition is administered said composition being prepared by mixing an alkaline powder with a powdered creatine to form a mixture with a pH in the range 7-14.

8. A method as claimed in claim 8 wherein the composition also is prepared from sweetener and/or taste enhancer.

9. Use of creatine and an alkaline powder in the preparation of compositions for treating, preventing or delaying endurance fatigue.

10. A use as claimed in claim 9 wherein the alkaline substance is selected from hydroxide, carbonate, bicarbonate, chloride, tree latex or a phosphate.

11. A use as claimed in claim 9 wherein the alkaline substance is selected from magnesium glyceryl phosphate and soda ash.

12. A use as claimed in claim 9 wherein substance is soda ash.

13. A use as claimed in any one of claims 9-12 wherein the creatine is in the form of the monohydrate, phosphate, pyruvate or citrate.

14. A use as claimed in any one of claims 9-13 wherein the composition is administered said composition being prepared by mixing an alkaline powder with a powdered creatine to form a mixture with a pH in the range 7-14.

15. A method for reducing the perception of fatigue in a subject during or after endurance exercise comprising administering to the subject a composition or compositions comprising creatine and an alkaline substance wherein the pH of the composition is in the range 7-14.

16. Use of creatine and an alkaline substance in the preparation of compositions for reducing the perception of fatigue in a subject during or after endurance exercise.
